# EUROPEAN PATENT SPECIFICATION

(11) **EP 3 189 872 B1**
(45) Date of publication and mention of the grant of the patent: **08.05.2019**
(21) Application number: 15838717.5
(22) Date of filing: 31.08.2015
(51) Int. Cl.: A61M 25/10, A61B 90/00

(54) **CATHETER**
KATHETER
CATHÉTER

(30) Priority: 04.09.2014 JP 2014180039; 04.09.2014 JP 2014180050; 04.09.2014 JP 2014180067
(43) Date of publication of application: 12.07.2017
(73) Proprietor: Terumo Kabushiki Kaisha, Tokyo 151-0072 (JP)
(72) Inventor: MAEDA, Naoyuki, Fujinomiya-shi Shizuoka 418-0015 (JP); SOUMA, Yuuki, Ashigarakami-gun Kanagawa 259-0151 (JP); TAKEUCHI, Ryota, Ashigarakami-gun Kanagawa 259-0151 (JP)
(74) Representative: Casalonga
(86) International application number: PCT/JP2015/074623
(87) International publication number: WO 2016/035739

(56) References cited:
- JP-A- 2008 253 800
- JP-A- 2014 518 739
- US-A1- 2010 042 199
- US-A1- 2011 082 489
- US-A1- 2011 172 698
- US-A1- 2013 261 547

## Description

### Technical Field

The present invention relates to a catheter including a balloon reinforced with a reinforcement member.

### Background Art

Recently, for example, in treatment of acute myocardial infarction and angina pectoris, percutaneous coronary intervention (percutaneous transluminal coronary angioplasty) in which a blood flow is improved by widening a lesion (stenosed portion) of the coronary artery with a balloon catheter has been performed (for example, refer to JP-T-2008-501408) . Treatment using a balloon catheter may be performed so as to improve a lesion formed inside other blood vessels, the bile duct, the trachea, the esophagus, the urethra, and other body lumens.

Generally, a balloon catheter is configured to include a long shaft, and a balloon which is provided on the distal side of the shaft and is inflated in the radial direction. The balloon catheter is delivered to a stenosed portion in a body after a preceding guide wire is inserted through. In a state where the balloon is disposed at the target stenosed site, the balloon is inflated by pressure-feeding a inflation fluid into the balloon, and thus, the stenosed portion can be widened.

In order to effectively treat a lesion area, the balloon used in the balloon catheter is required to have sufficient strength so as to have a desired balloon shape when being maximally inflated, and to widen the lesion. Therefore, in the related art, in order to apply high-pressure resistance, low compliance properties, and the like to a balloon, a configuration in which a net-shaped reinforcement member is provided in a wall configuring the balloon has been proposed (for example, refer to JP-T-2008-501408). An example of a balloon catheter according to the preamble of claim 1 is disclosed in US 2011/082489.

### Summary of Invention

In addition, a balloon catheter transports a balloon to a lesion inside a body lumen. Since the balloon needs to pass through the inside of the bent body lumen while being transported, the balloon is required to have flexibility so as to follow the bent state of the body lumen. However, the technology in the related art, in which a reinforcement member is provided in a wall configuring a balloon, has a problem in that since the reinforcement member is integrally fixed to the balloon and there is no degree of freedom for movement with respect to the wall of the balloon, it is difficult to apply sufficient flexibility to the balloon.

The present invention has been made in consideration of such a problem, and an object thereof is to provide a catheter in which flexibility of a balloon reinforced with a reinforcement member can be improved.

In order to achieve the above-described object, according to the present invention, there is provided a catheter including a balloon that has an inner layer and an outer layer having elastic stretching properties, having tubular shapes, and being able to be inflated and deflated in response to a change of internal pressure; and a tubularly net-shaped reinforcement member that is disposed between the inner layer and the outer layer. The reinforcement member has a first end portion and a second end portion in an axial direction, and an intermediate portion configuring a portion between the first end portion and the second end portion. At least one of the first end portion and the second end portion, and the intermediate portion are not directly fixed to the inner layer and the outer layer. Here, the expression "at least one of the first end portion and the second end portion, and the intermediate portion are not directly fixed to the inner layer and the outer layer" denotes that at least one of the first end portion and the second end portion, and the intermediate portion are not bonded to the inner layer and the outer layer and are not embedded in the inner layer and the outer layer, thereby being able to freely move inside a space formed between the inner layer and the outer layer.

According to the configuration, substantially the entirety of the reinforcement member has the degree of freedom for moving in the axial direction and a circumferential direction with respect to the balloon. Therefore, favorable flexibility of the balloon can be maintained. Accordingly, crossability of the catheter inside a body lumen can be improved. In addition, the balloon has the configuration in which the reinforcement member is disposed between the inner layer and the outer layer having elastic stretching properties. Therefore, high-pressure resistance and low compliance properties can be suitably applied. Here, the term low compliance properties denotes characteristics in which when the balloon is inflated under high pressure, a balloon diameter is not unlimitedly widened in response to the pressure, and inflation under high pressure can be performed in an appropriate diameter. Moreover, the balloon is inflated and deflated while entailing elastic stretching and is a zero folding-type balloon which is not folded when being in a deflated state. Accordingly, the balloon can easily restore the original outer diameter when being deflated again after inflation. Therefore, crossability inside a body lumen can be further improved. Furthermore, the balloon having elastic stretching properties can be prepared without performing blow molding. Therefore, the catheter can be conveniently manufactured.

In the catheter, the other of the first end portion and the second end portion may not be also directly fixed to the inner layer and the outer layer. According to the configuration, the reinforcement member is not fixed to any one of the inner layer and the outer layer. Therefore, the degree of freedom for moving the reinforcement member with respect to the inner layer and the outer layer of the balloon can be further improved, and flexibility with respect to bending can be improved. In accordance therewith, crossability inside a body lumen can be further improved.

In the catheter, inflation of the first end portion and the second end portion in the circumferential direction may be restricted. According to the configuration, in the reinforcement member, the maximally inflated diameter of the intermediate portion positioned between the first end portion and the second end portion can be effectively restricted. Therefore, the function as a reinforcement member can be suitably conducted.

In the catheter, the reinforcement member may be formed by tubularly knitting one or more threads, and the waved threads adjacent to each other in the axial direction may be interlaced with each other. According to the configuration, when the reinforcement member is compressed in the circumferential direction, the threads are folded in the circumferential direction, and when the reinforcement member is compressed in the axial direction, the threads of the meshes are misaligned in the axial direction. Therefore, the reinforcement member can be flexibly bent.

In the catheter, the reinforcement member may be formed of a high-strength fiber of which tensile break strength is equal to or greater than 2 GPa and an elastic modulus is equal to or greater than 50 GPa. According to the configuration, it is possible to realize a balloon having excellent high-pressure resistance and low compliance properties.

In the catheter, an axial-directional length of the reinforcement member may be greater than an axial-directional length of a region in the inner layer that is stretchable during deformation of inflation and deflation of the balloon. An axial-directional length of the outer layer may be greater than the axial-directional length of the reinforcement member. According to the configuration, due to the reinforcement member which is disposed between the inner layer and the outer layer while having an appropriate length, the maximally inflated diameters of the inner layer and the outer layer can be reliably restricted.

In the catheter, a joint structure that partially joins the inner layer and the outer layer to each other via multiple gaps penetrating inner and outer surfaces of the reinforcement member and that is not fixed to the reinforcement member may be provided such that the reinforcement member may be restrained from being misaligned in the axial direction with respect to the balloon. According to the configuration, the reinforcement member is restrained from being misaligned in the axial direction with respect to the balloon. Therefore, a state of the balloon reinforced by the reinforcement member can be suitably maintained. Therefore, even after inflation and deflation of the balloon are repeated multiple times, the balloon is not ruptured while being under low pressure. Therefore, the catheter can be stably used.

In the catheter, the joint structure may have multiple welded portions respectively penetrating the multiple gaps. The reinforcement member may be movable with respect to the welded portions. According to the configuration, the wire member is caught by the welded portion. Therefore, the reinforcement member is appropriately restrained from being misaligned. Therefore, it is possible to simply realize a structure in which the reinforcement member is effectively restrained from being misaligned in the axial direction, without fixing the reinforcement member to the inner layer and the outer layer through the joint structure.

In the catheter, the joint structure may be provided in only a region of the reinforcement member on the first end portion side and a region of the reinforcement member on the second end portion side. According to the configuration, while achieving favorable flexibility of the balloon, the reinforcement member can be effectively restrained from being misaligned in the axial direction.

In the catheter, the multiple gaps may be meshes in the reinforcement member. According to the configuration, the meshes in the reinforcement member can be utilized. Therefore, there is no need to provide a dedicated gap for joining the inner layer and the outer layer to each other.

In the catheter, the reinforcement member may be formed so as to include one or more wire members. A lubricant may be present on at least an outer surface of the wire member such that friction between portions of the wire members in contact with each other may be reduced. According to the configuration, friction between the portions of the wire members in contact with each other can be reduced, and the degree of freedom of movement between the wire members can be enhanced. Therefore, when being deflated again after inflation, the balloon can easily restore the original shape (thickness) before being inflated.

In the catheter, the lubricant may be applied to the wire member. According to the configuration, the amount of a using lubricant can be restrained, and friction between the portions of the wire members in contact with each other can be efficiently reduced.

In the catheter, an accommodation chamber accommodating the reinforcement member may be formed between the inner layer and the outer layer. The accommodation chamber may be filled with the lubricant. According to the configuration, a state where a lubricant is present around the wire member configuring the reinforcement member can be maintained. Therefore, friction between the portions of the wire members in contact with each other can be reliably reduced.

In the catheter, the wire member may be formed of a high-strength fiber of which tensile break strength is equal to or greater than 2 GPa and an elastic modulus is equal to or greater than 50 GPa. Generally, frictional resistance between high-strength fibers (super fibers) is high. Therefore, in a case where the reinforcement member is configured with the high-strength fiber, if the fibers are in direct contact with each other without being lubricated, there is a possibility that when the balloon is deflated again, it may be difficult for the balloon to restore the original shape before being inflated. In contrast, when the configuration in which a lubricant is present on the outer surface of the wire member (thread) formed of the high-strength fiber is employed, a problem of frictional resistance occurring when a high-strength fiber is used as the wire member can be restrained, and high-pressure resistance and low compliance properties can be effectively applied to the balloon.

In addition, according to the present invention, there is provided a catheter which includes a balloon that has an inner layer and an outer layer having elastic stretching properties, having tubular shapes, and being able to be inflated and deflated in response to a change of internal pressure, and a reinforcement member that is disposed between the inner layer and the outer layer and has a tubularly net-shaped structure; and in which the reinforcement member has a first end portion and a second end portion in an axial direction, and an intermediate portion configuring a portion between the first end portion and the second end portion. The catheter manufacturing method which does not form part of the invention includes a disposition step of disposing the reinforcement member between the inner layer and the outer layer, and a joint step of joining one end portion of the inner layer and one end portion of the outer layer to each other and the other end portion of the inner layer and the other end portion of the outer layer to each other without directly fixing at least one of the first end portion and the second end portion, and the intermediate portion to the inner layer and the outer layer.

According to the catheter manufacturing method, it is possible to manufacture a catheter which includes a balloon having high flexibility as well as high-pressure resistance and low compliance properties.

In the catheter manufacturing method, the catheter manufacturing method may further include a welding step of partially welding the inner layer and the outer layer to each other via a gap penetrating inner and outer surfaces of the reinforcement member without fixing the reinforcement member to the inner layer and the outer layer. Accordingly, there is provided a structure in which the reinforcement member is restrained from being misaligned in the axial direction with respect to the inner layer and the outer layer. Therefore, even after inflation and deflation are repeated multiple times, the balloon is not ruptured while being under low pressure.

In the catheter manufacturing method, the catheter manufacturing method may further include a step of providing the reinforcement member which is wetted with liquid. In the welding step, in a state where the reinforcement member wetted with the liquid is disposed between the inner layer and the outer layer, the inner layer and the outer layer may be heated and be welded to each other. Accordingly, in the welding step, the reinforcement member wetted with the liquid is not welded to the inner layer and the outer layer. Thus, it is possible to simply form a structure in which even though the inner layer and the outer layer are partially welded to each other, the reinforcement member is movable with respect to the inner layer and the outer layer.

In the catheter manufacturing method, in the welding step, one or more regions in each of the inner layer and the outer layer in the axial direction may be selectively heated. Accordingly, while achieving favorable flexibility of the balloon, the reinforcement member can be effectively restrained from being misaligned in the axial direction.

In the catheter manufacturing method, in the welding step, only a spot of the gap in the reinforcement member may be irradiated with a laser such that the inner layer and the outer layer may be welded to each other. Accordingly, without wetting the reinforcement member with liquid, the inner layer and the outer layer can be welded to each other at a spot avoiding the wire member.

In the catheter manufacturing method, in the welding step, the entirety of the balloon may be heated. Accordingly, the welding step can be efficiently carried out.

According to the present invention, in the catheter flexibility of the balloon reinforced with the reinforcement member can be improved.

### Brief Description of Drawings

Fig. 1 is a partially-omitted schematic view illustrating a configuration of a catheter, according to a first embodiment of the present invention.
Fig. 2 is a schematic cross-sectional view of the distal portion of the catheter illustrated in Fig. 1.
Fig. 3A is a side view illustrating a reinforcement member when being inflated, and Fig. 3B is a side view illustrating the reinforcement member when being deflated.
Fig. 4A is a view describing a step of preparing a base material sleeve, and Fig. 4B is a view describing a step of preparing multiple reinforcement members from the base material sleeve.
Fig. 5A is a view describing a step of covering an inner layer tube with the reinforcement member, and Fig. 5B is a view describing a step of covering the inner layer tube and the reinforcement member with an outer layer tube.
Fig. 6A is a first view describing a step of joining the inner layer tube and the outer layer tube to each other, and Fig. 6B is a second view describing the step of joining the inner layer tube and the outer layer tube to each other.
Fig. 7A is a first view describing a step of joining the distal end of a shaft and the proximal end of a balloon to each other, and Fig. 7B is a second view describing the step of joining the distal end of the shaft and the proximal end of the balloon to each other.
Fig. 8A is a first view describing a step of joining an inner tube and the distal end of the balloon to each other, and Fig. 8B is a second view describing the step of joining the inner tube and the distal end of the balloon to each other.
Fig. 9A is a first view describing a step of joining a distal tip and the inner tube to each other, and Fig. 9B is a second view describing the step of joining the distal tip and the inner tube to each other.
Fig. 10 is a graph illustrating a relationship between pressure and a balloon diameter regarding balloons having different forms of fixing the reinforcement member from each other, and a balloon provided with no reinforcement member.
Fig. 11 is a schematic cross-sectional view of the distal portion of a catheter according to a second embodiment of the present invention.
Fig. 12 is a schematic view in which a joint structure of an inner layer and an outer layer is developed in a circumferential direction.
Fig. 13 is a view describing a step of wetting the reinforcement member.
Fig. 14A is a first view describing a step of forming the joint structure, and Fig. 14B is a second view describing the step of forming the joint step.
Fig. 15 is a schematic cross-sectional view of the distal portion of a catheter according to a modification example of the second embodiment.
Fig. 16 is a schematic cross-sectional view of the distal portion of a catheter according to another modification example of the second embodiment.
Fig. 17 is a schematic cross-sectional view of the distal portion of a catheter according to a third embodiment of the present invention.
Fig. 18 is a view describing a step of applying a lubricant to threads configuring the reinforcement member.
Fig. 19 is a schematic cross-sectional view of the distal portion of a catheter according to a modification example of the third embodiment.

### Description of Embodiments

Hereinafter, a catheter according to the present invention will be described based on multiple preferable embodiments with reference to the accompanying drawings. Note that, regarding second and third embodiments and modification examples thereof, the same reference signs will be applied to elements which are the same as or similar to those of a first embodiment, and detailed description will be omitted.

### [First Embodiment]

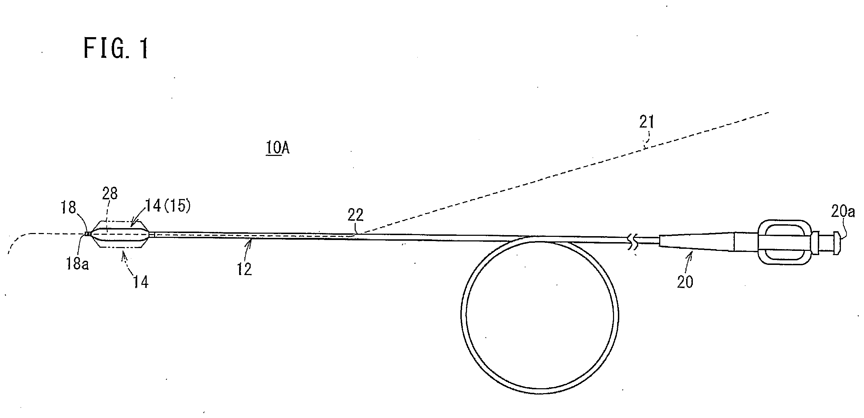

Fig. 1 is a partially-omitted schematic view illustrating a configuration of a catheter 10A, according to the first embodiment of the present invention. The catheter 10A is a so-called PTCA (percutaneous transluminal coronary angioplasty: percutaneous coronary intervention) inflation catheter for performing treatment in which a long shaft 12 is inserted through a biological organ, for example, the coronary artery, a balloon 14 provided on the distal side thereof is inflated at a stenosed portion (lesion), and the stenosed portion is widened.

The present invention can also be applied to a catheter other than the PTCA inflation catheter, for example, a catheter for improving a lesion formed inside biological organs such as other blood vessels, the bile duct, the trachea, the esophagus, the urethra, and other internal organs.

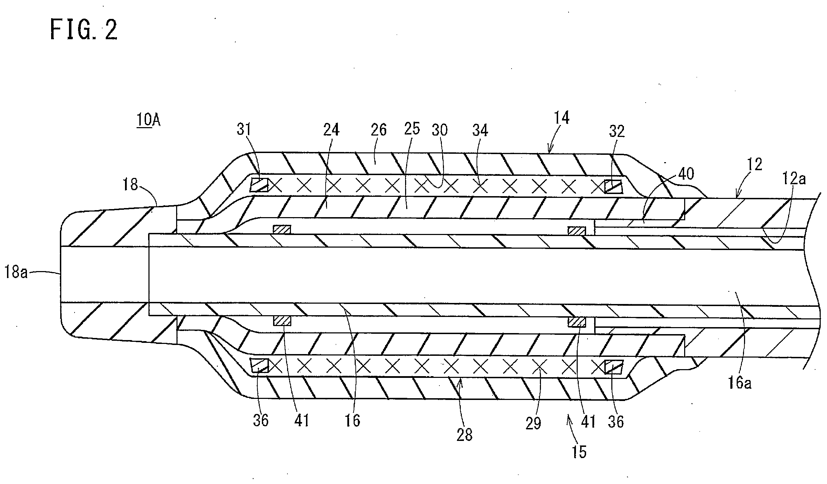

As illustrated in Fig. 1 (and Fig. 2), the catheter 10A includes the long shaft 12 having a small diameter, the balloon 14 joined to the distal end of the shaft 12, a reinforcement member 28 disposed inside a membrane (wall) configuring the balloon 14, an inner tube 16 inserted through the shaft 12 and the balloon 14, a distal tip 18 joined to the distal end of the balloon 14, and a hub 20 provided on the proximal side of the shaft 12.

In Fig. 1, the catheter 10A is configured as a so-called "rapid exchange-type" catheter provided with an opening portion 22 through which a guide wire 21 is guided out, in a middle portion of the shaft 12 in a longitudinal direction. In another embodiment, the catheter 10A may be configured as an "over-the-wire-type" catheter in which a guide wire lumen is formed throughout the overall length of the catheter 10A, and the guide wire 21 is guided out from the proximal end of the hub 20.

The shaft 12 is a flexible tube of which both ends in an axial direction are open, and which is long and has a small diameter. The shaft 12 extends from the rear end of the balloon 14 to the distal end of the hub 20. A portion from the distal end to the opening portion 22 configures a double tube which forms a inflation lumen 12a between the shaft 12 and the inner tube 16, and a portion from the opening portion 22 to the hub 20 is a single tube. The shaft 12 forms the inflation lumen 12a through which a inflation fluid for the balloon 14 is supplied.

In the shaft 12, the inflation fluid pressure-fed from a pressure applying apparatus such as an indeflator (not illustrated) connected via a luer taper 20a or the like provided in the hub 20 can be fed to the balloon 14. For example, the inflation fluid is a contrast agent, a physiological salt solution, or a mixture thereof.

The inner tube 16 is a guide wire tube forming a wire lumen 16a through which the guide wire 21 is inserted. The distal end of the inner tube 16 is positioned on the distal side beyond the proximal end of the distal tip 18. The inner tube 16 extends inside the balloon 14 and the shaft 12, and the proximal end thereof is liquid-tightly joined to the opening portion 22 (refer to Fig. 1) formed in an intermediate portion of the shaft 12. Therefore, the guide wire 21 which has inserted through a distal end opening portion 18a serving as an entrance at the distal tip 18 is inserted through the wire lumen 16a of the inner tube 16 from the distal side toward the proximal side and is guided out through the opening portion 22 serving as an exit.

It is favorable to provide a radiopaque marker 41 in the inner tube 16 inside the balloon 14. The radiopaque marker 41 is configured with an X-ray opaque (radiopaque) material (for example, gold, platinum, tungsten, or a mixture thereof) . The radiopaque marker 41 is used for visually recognizing the position of the balloon 14 in a living body under an X-ray contrast condition. For example, the radiopaque marker 41 can be configured to have a tubular shape (ring shape) . Note that, as in Fig. 2, multiple imaging markers 41 may be provided on the inner tube 16 inside the balloon 14 while being spaced from each other in the axial direction. Otherwise, only one radiopaque marker 41 may be provided on the inner tube 16 inside the balloon 14.

It is preferable that the shaft 12 and the inner tube 16 have structures with appropriate flexibility and appropriate rigidity such that an operator can smoothly insert the long catheter 10A into a biological organ such as a blood vessel while grasping and operating the proximal side of the catheter 10A. Therefore, for example, it is favorable that the shaft 12 and the inner tube 16 are formed of a polymeric material such as polyolefin (for example, polyethylene, polypropylene, polybutene, an ethylene-propylene copolymer, an ethylene-vinyl acetate copolymer, ionomer, and a mixture of two or more types thereof), polyvinyl chloride, polyamide, a polyamide elastomer, polyurethane, a polyurethane elastomer, polyimide, a fluorine resin, and a mixture thereof; or a a multi-layer tube including two or more types thereof.

The balloon 14 can be inflated and dilated in response to a change of internal pressure. The distal portion of the balloon 14 is joined to a portion in the vicinity of the distal portion of the inner tube 16, and the proximal portion of the balloon 14 is joined to the distal portion of the shaft 12. The internal space of the balloon 14 communicates with the inflation lumen 12a.

Via the inflation lumen 12a, the inflation fluid can flow into (be guided into) the balloon 14 and the inflation fluid can be discharged from the balloon 14. In response to the inflation fluid guided into the balloon 14, the balloon 14 is inflated. As indicated with the imaginary line in Fig. 1, when the balloon 14 is maximally inflated, a portion between the distal end and the proximal end exhibits a shape which is increased in diameter and has a substantially uniform outer diameter along the axial direction.

The balloon 14 is required to have appropriate flexibility so as to be able to pass through a meandering or bent point of a body lumen. In addition, the balloon 14 is required to have strength to the extent that a lesion can be reliably widened, and the balloon 14 is also required to have high-pressure resistance and low compliance properties. Therefore, in the present embodiment, the balloon 14 has an inner layer 24 and an outer layer 26 having tubular shapes, having elastic stretching properties, and configuring fluid-impermeable balloon walls. The reinforcement member 28 is disposed between the inner layer 24 and the outer layer 26. The balloon 14 and the reinforcement member 28 configure a dilation portion 15 which can be inflated and deflated in a radial direction at the distal portion of the catheter 10A.

The inner layer 24 transfer force to the reinforcement member 28 in response to the inflation fluid guided into the balloon 14 (pressurization), and the inner layer 24 expands to the extent of a shape which is restricted along the dilated shape of the reinforcement member 28. The outer layer 26 swells along the inflated shape of the reinforcement member 28 in response to the inflation fluid guided into the balloon 14 (pressurization), and the outer layer 26 contracts to the extent of the initial shape in response to the inflation fluid discharged from the inside of the balloon 14 (decompression) in order to restore the original shape (position) of the reinforcement member 28. Therefore, it is preferable that the outer layer 26 is formed of a material having a high stretching recovery rate.

The inner layer 24 and the outer layer 26 are joined to each other at the distal portions and the proximal portions, for example, through welding or bonding. An annularly sealed accommodation chamber 30 accommodating the reinforcement member 28 is formed between the inner layer 24 and the outer layer 26.

Examples of the configuring materials of the inner layer 24 and the outer layer 26 include various types of rubber material such as natural rubber, butyl rubber, isoprene rubber, butadiene rubber, styrene-butadiene rubber, and silicone rubber; various types of thermoplastic elastomer such as a polyurethane-based elastomer, a polyester-based elastomer, a polyamide-based elastomer, an olefin-based elastomer, and a styrene-based elastomer; mixtures thereof; and the like. The configuring material of the inner layer 24 and the configuring material of the outer layer 26 may be the same as each other or may be different from each other.

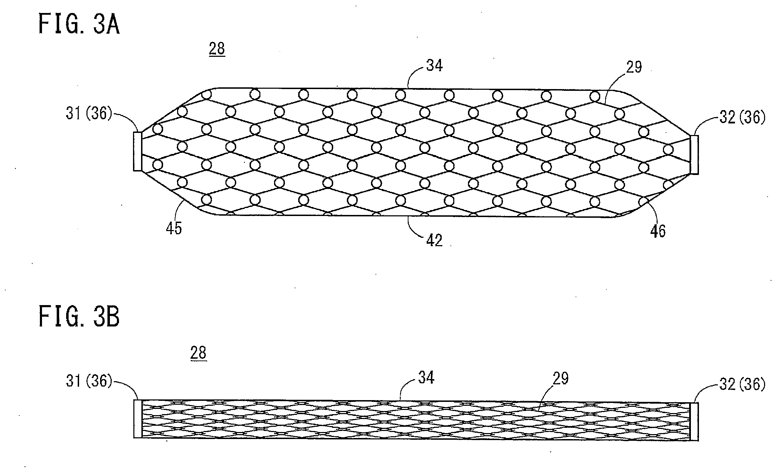

The reinforcement member 28 is a tubularly net-shaped member configuring an intermediate layer between the inner layer 24 and the outer layer 26, and the reinforcement member 28 functions to enhance pressure resistance of the balloon 14. The reinforcement member 28 is formed so as to have a tubularly net-shaped body by knitting (weaving) one or more threads 29, and the reinforcement member 28 has stretching properties in at least a circumferential direction (and the radial direction) . The method of forming the reinforcement member 28 is not limited to any particular form. Examples of the method include tube-knitting and braiding. In a case of tube-knitting, in the reinforcement member 28, the threads 29 extending in the circumferential direction in a waved manner are arranged in the axial direction, and the waved threads 29 adjacent to each other in the axial direction are interlaced with each other (refer to Fig. 3A). In a case of braiding, in the reinforcement member 28, one or more threads 29 extending in a first spiral direction and one or more s threads 29 extending in a second spiral direction are woven so as to intersect each other, thereby forming the tubularly net-shaped body.

In order to apply high-pressure resistance and low compliance properties to the balloon 14, as the thread 29 configuring the reinforcement member 28, it is preferable to employ the thread 29 having high strength and a high elastic modulus, for example, a twisted thread formed of high-strength fibers (super fibers) of which tensile break strength is equal to or greater than 2 GPa and an elastic modulus is equal to or greater than 50 GPa. Examples of the high-strength fiber include an aramid fiber, a carbon fiber, a polyarylate fiber, a PBO fiber, ultra-high molecular weight polyethylene, and an LCP fiber.

For example, the diameter of the thread 29 may range approximately from 5 to 100 µm. In a case where the twisted thread formed of the high-strength fibers is used as the thread, for example, a single fiber diameter of the high-strength fiber may range approximately from 5 to 30 µm. As the high-strength fiber, for example, a fiber having a single fiber diameter of 12 µm can be used. However, a thinner fiber may be used, and a thicker fiber may be used. In a case of a thicker fiber, it is favorable to employ a loosely twisted thread so as to be in an unraveled state when tensile force is not applied to the twisted thread.

The reinforcement member 28 has both end portions (first end portion 31 and second end portion 32) in the axial direction, and an intermediate portion 34 configuring a portion between the first end portion 31 and the second end portion 32. In the reinforcement member 28, at least one of the first end portion 31 and the second end portion 32, and the intermediate portion 34 are not directly fixed to the inner layer 24 and the outer layer 26. Accordingly, movement with respect to the inner layer 24 and the outer layer 26 in the axial direction and the circumferential direction is allowed.

In addition, the inner layer 24 and the outer layer 26 may be fixedly attached (for example, welded or bonded) via a gap (mesh) between the threads 29 forming the reinforcement member 28. Accordingly, while the reinforcement member 28 is allowed to move with respect to the inner layer 24 and the outer layer 26 to a certain extent, the moving range of the reinforcement member 28 can be restricted.

In a case of the present embodiment, the other one of the first end portion 31 and the second end portion 32 is not also directly fixed to the inner layer 24 and the outer layer 26. Accordingly, movement with respect to the inner layer 24 and the outer layer 26 in the axial direction is allowed. In other words, in the present embodiment, the reinforcement member 28 is not fixed to any one of the inner layer 24 and the outer layer 26. Therefore, the reinforcement member 28 can freely move in the circumferential direction and the axial direction within a range restricted by the inner layer 24 and the outer layer 26 (within a range of the accommodation chamber 30) .

Note that, only one of the first end portion 31 and the second end portion 32 may be fixed to the inner layer 24 or the outer layer 26. In this case, fixing means is not limited to any particular means and suitable fixing means such as welding and bonding may be employed.

Inflation of the first end portion 31 and the second end portion 32 of the reinforcement member 28 in the circumferential direction and the radial direction is restricted by an inflation restriction portion 36. The inflation restriction portion 36 is not limited to a particular configuration. Example of the inflation restriction portion 36 include configurations such as a knitted portion in which inflation in the circumferential direction and the radial direction is restricted through a knitting method, ring-shaped welded portions which are formed at the first end portion 31 and the second end portion 32 by welding threads (fibers) having welding properties to each other, and ring-shaped retention members which are respectively fixed to the first end portion 31 and the second end portion 32.

As illustrated in Fig. 2, the distal portion of the inner layer 24 is joined to the inner tube 16.

In addition, the proximal portion of the inner layer 24 is joined to the distal portion (thin portion 40) of the shaft 12, and the outermost distal portion of the shaft 12 is positioned on the distal side beyond the innermost proximal surface of the inner layer 24, on the inner side of the inner layer 24. Therefore, a region (hereinafter, will be referred to as "stretchable region 25 of the inner layer 24") in the inner layer 24 that is stretchable during deformation of inflation and deflation of the balloon 14 is a portion between a joint spot of the inner layer 24 and the inner tube 16, and the outermost distal portion of the shaft 12.

The innermost proximal portion of the reinforcement member 28 is positioned on the proximal side beyond the innermost proximal portion of the stretchable region 25 in the inner layer 24. Note that, as illustrated in Fig. 2, the second end portion 32 of the reinforcement member 28 may be disposed on the proximal side beyond the outermost distal portion of the shaft 12 in the accommodation chamber 30 formed between the inner layer 24 and the outer layer 26 of the balloon 14. Accordingly, when the balloon 14 is inflated, the second end portion 32 of the reinforcement member 28 is less affected by inflation of the balloon 14 and contributes to restriction of the maximally inflated diameter of the balloon 14 performed by the reinforcement member 28.

Fig. 3A is a side view illustrating the reinforcement member 28 when being inflated, and Fig. 3B is a side view illustrating the reinforcement member 28 when being deflated. As illustrated in Fig. 3A, when the reinforcement member 28 is inflated in the circumferential direction, the threads 29 are in a tensed state, and the outer diameter thereof is not increased to a certain extent or greater. In this case, since inflation of the first end portion 31 and the second end portion 32 is restricted, the shape of the reinforcement member 28 (intermediate portion 34) when being inflated includes a straight portion 42 having a substantially uniform outer diameter, and outer-diameter varying portions (tapered portions) 45 and 46 which are respectively positioned on both sides of the straight portion 42 and are decreased in diameter outward in the axial direction.

Note that, in a case where the balloon 14 is produced by using the reinforcement member 28 illustrated in Figs. 3A and 3B, the balloon 14 when being inflated includes a straight portion having a substantially uniform outer diameter due to the reinforcement member 28, and outer-diameter varying portions (tapered portions) which are respectively positioned on both sides of the straight portion and are decreased in diameter outward in the axial direction. In such a case, the radiopaque marker 41 is disposed on the inner tube 16 such that the position of the straight portion of the balloon 14 can be confirmed. Accordingly, since an operator can visually recognize the position having the maximally inflated diameter in the balloon 14 under an X-ray contrast condition, positioning between the region of the maximally dilated diameter in the balloon 14, and the lesion can be easily performed.

In a case of the reinforcement member 28 formed through the knitting method in which the waved threads 29 adjacent to each other in the axial direction are interlaced with each other, when the reinforcement member 28 is compressed in the circumferential direction as illustrated in Fig. 3B, the threads 29 are folded and the reinforcement member 28 is decreased in diameter. In addition, when the reinforcement member 28 is compressed in the axial direction, the threads 29 of the meshes are misaligned and the threads 29 adjacent to each other in the axial direction can overlap each other. Moreover, the reinforcement member 28 can be bent in accordance with rotations of the interlaced portion between the threads 29 adjacent to each other in the axial direction. Therefore, such a reinforcement member 28 has excellent flexibility with respect to bending.

In Figs. 1 and 2, the distal tip 18 provided on the distal side of the balloon 14 is a portion which flexibly advances through a curved portion, an irregular portion, and the like inside a biological organ, as the outermost distal end of the catheter 10A, penetrates a lesion (stenosed portion), and leads the catheter 10A to be smoothly inserted through. The distal tip 18 is a short tube having an inner diameter substantially the same as the inner diameter of the inner tube 16.

The distal tip 18 is fitted to the distal portion of the inner tube 16 from the outside so as to be liquid-tightly joined to the distal portion of the inner tube 16. The distal tip 18 protrudes toward the distal side beyond the distal end opening portion 18a of the wire lumen 16a, and the proximal surface thereof is joined to the distal surface of the balloon 14. The distal end opening portion 18a of the distal tip 18 communicates with the wire lumen 16a of the inner tube 16 and serves as the entrance of the guide wire 21.

The material and the shape of the distal tip 18 are suitably set such that the distal tip 18 is configured to be more flexible than at least the shaft 12 and the inner tube 16. Note that, the distal tip 18 may be omitted. In such a case, it is favorable to employ a configuration in which the outermost distal end position of the inner tube 16 and the outermost distal end position of the balloon 14 coincide with each other, or a configuration in which the outermost distal end position of the inner tube 16 slightly protrudes beyond the outermost distal end position of the balloon 14.

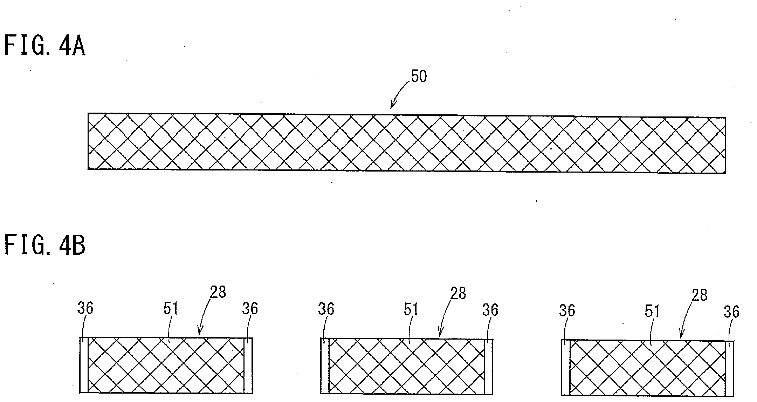

Subsequently, an example of a method of manufacturing the catheter 10A (mainly, a step of manufacturing the inflation portion 15 and peripheral portions thereof) will be described. In Figs. 4A to 9B, the tubularly net-shaped reinforcement member 28 is schematically illustrated, and the reinforcement member 28 is not limited to any particular knitting method.

Figs. 4A and 4B are views describing the step of manufacturing the reinforcement member 28. As in Fig. 4A, first, a step of preparing a tubularly net-shaped base material sleeve 50 which becomes the base material of the reinforcement member 28 (step of preparing a base material sleeve) is executed. The base material sleeve 50 has a length equal to or greater than those of multiple reinforcement members 28. In this case, for example, the base material sleeve 50 is formed by knitting the above-described high-strength fibers so as to have a tubular net shape.

Subsequently, as in Fig. 4B, the base material sleeve 50 is cut at one or more spots in the axial direction so as to have multiple divided sleeves 51, and a step of forming the above-described inflation restriction portion 36 (cutting and restriction processing step) is executed at both end portions of each divided sleeve 51. Accordingly, multiple reinforcement members 28 are obtained.

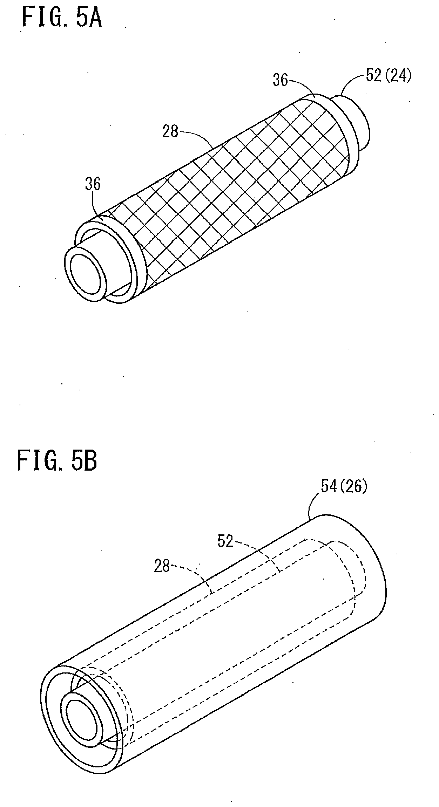

Subsequently, as in Fig. 5A, a step of covering an inner layer tube 52 which is the base material of the inner layer 24, with the reinforcement member 28 (first covering step) is executed. In this case, both end portions of the inner layer tube 52 respectively protrude beyond openings at both ends of the reinforcement member 28.

Subsequently, as in Fig. 5B, a step of covering the inner layer tube 52 and the reinforcement member 28 (reinforcement member 28 in a state where the inner layer tube 52 is inserted into the inner side) with an outer layer tube 54 which is the base material of the outer layer 26 (second covering step) is executed. In this case, the inner layer tube 52 and the reinforcement member 28 are covered with the outer layer tube 54 such that the overall length of the reinforcement member 28 is housed inside the outer layer tube 54 (both end portions of the outer layer 26 protrude in the axial direction beyond both the end portions of the reinforcement member 28).

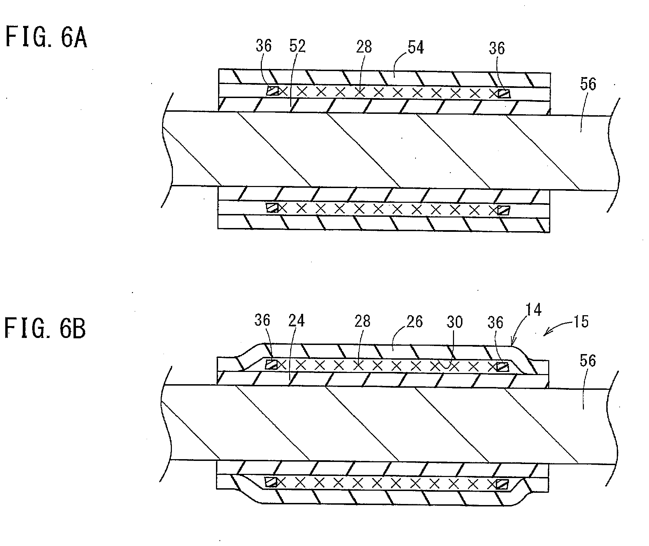

Subsequently, a step of joining the inner layer tube 52 and the outer layer tube 54 (step of joining inner and outer layers) is executed. Specifically, first, as in Fig. 6A, a mandrel 56 (core bar) is inserted into the inner layer tube 52 (assembly of the inner layer tube 52, the outer layer tube 54, and the reinforcement member 28). Subsequently, as in Fig. 6B, one end portion of the inner layer tube 52 and one end portion of the outer layer tube 54 are welded so as to be joined to each other, and the other end portion of the inner layer tube 52 and the other end portion of the outer layer tube 54 are welded so as to be joined to each other. Accordingly, the annularly sealed accommodation chamber 30 is formed between the inner layer 24 and the outer layer 26, thereby obtaining the inflation portion 15 in a state where the reinforcement member 28 is disposed inside the accommodation chamber 30. After the step of joining inner and outer layers, the mandrel 56 is removed.

In this case, in the present embodiment, the reinforcement member 28 is merely disposed inside the accommodation chamber 30 and is not joined to other members through welding, bonding, or the like. Therefore, the reinforcement member 28 is not fixed to any portion of the balloon 14 (inner layer 24 and outer layer 26).

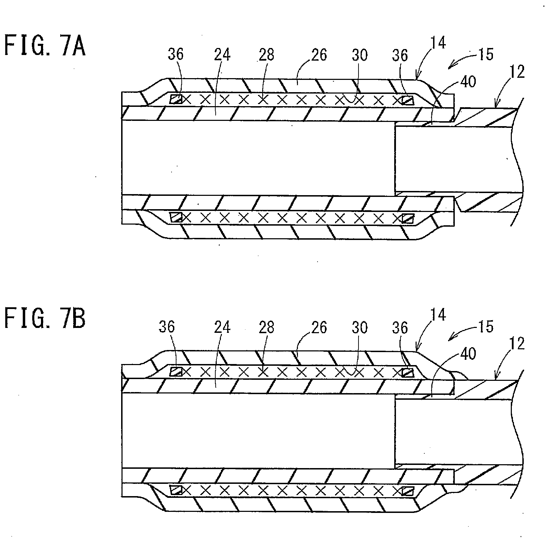

Subsequently, a step of joining the balloon 14 (inflation portion 15) and the shaft 12 to each other (step of joining a balloon and a shaft) is executed (Figs. 7A and 7B). Specifically, the thin portion 40 is formed at the distal portion of the shaft 12. In this case, for example, the distal portion of the shaft 12 is drawn down (the mandrel is inserted into a hollow portion of the shaft 12, and the distal portion of the shaft 12 is pressedly input into a mold which includes a hole having a diameter smaller than that of the shaft 12), and thus, the distal portion can have a small diameter. Subsequently, as in Fig. 7A, the thin portion 40 of the shaft 12 is inserted into the proximal side of the balloon 14. Subsequently, as in Fig. 7B, the proximal portion of the balloon 14 and the distal portion (thin portion 40) of the shaft 12 are welded so as to be joined to each other.

Subsequently, even though the step is not illustrated, the radiopaque marker 41 is attached to the inner tube 16. Specifically, the tubular radiopaque marker 41 having an inner diameter slightly greater than the inner tube 16 is caused to pass through the outer side of the inner tube 16, and the mandrel is inserted into the inner tube 16. Thereafter, the entire circumference of the radiopaque marker 41 is beaten (swaging step) . The radiopaque marker 41 is decreased in diameter and is engaged with the inner tube 16. In this manner, the imaging marker 41 is fixed to the inner tube 16.

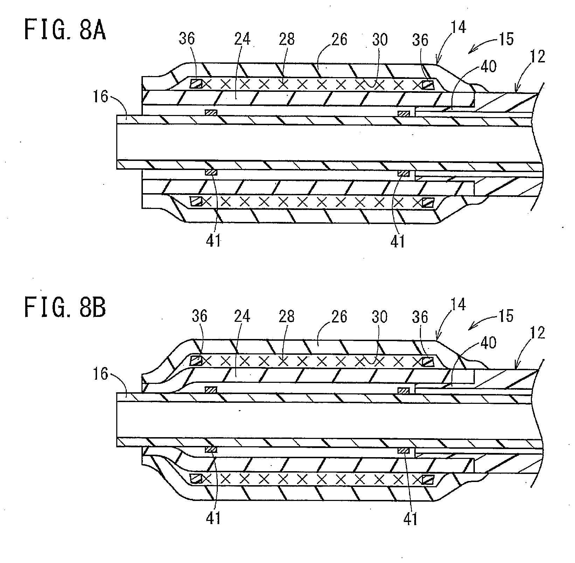

Subsequently, a step of joining the balloon 14 and the inner tube 16 to each other (step of joining a balloon and an inner tube) is executed (Figs. 8A and 8B). Specifically, as in Fig. 8A, the inner tube 16 is inserted into the balloon 14 and the shaft 12. Subsequently, as in Fig. 8B, the distal portion of the balloon 14 and the inner tube 16 are welded so as to be joined to each other.

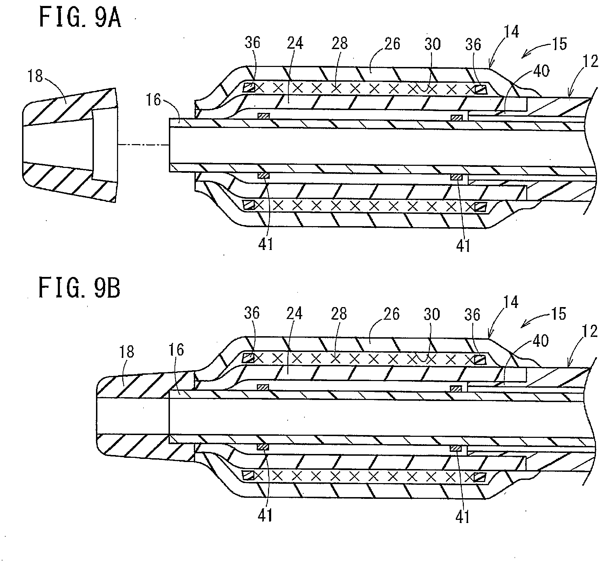

Subsequently, a step of joining the distal tip 18 and the inner tube 16 to each other (step of joining a distal tip and an inner tube) is executed (Figs. 9A and 9B). Specifically, first, the distal portion of the inner tube 16 is cut, and the length is adjusted (Fig. 9A). Subsequently, the proximal portion of the distal tip 18 is fitted to the distal portion of the inner tube 16 from the outside, and the proximal portion of the distal tip 18 and the distal portion of the inner tube 16 are welded so as to be joined to each other (Fig. 9B).

Note that, a step of joining the proximal end of the shaft 12 and the distal portion of the hub 20 to each other (step of joining a shaft and a hub) can be executed at an arbitrary time. For example, the step of joining a shaft and a hub may be executed before the step of joining a balloon and a shaft, may be executed after the step of joining a distal tip and an inner tube, or may be executed between the step of joining a balloon and a shaft and the step of joining a distal tip and an inner tube.

In the above-described manufacturing method, as the means for joining members to each other, welding is exemplified. However, instead of welding, other types of joint means such as bonding may be applied.

The catheter 10A according to the present embodiment is basically configured as described above. Hereinafter, operations and effects thereof will be described.

For example, treatment using the catheter 10A is performed as follows. First, a form of a lesion (stenosed portion) occurred inside a blood vessel is specified through an intravascular contrast method or an intravascular ultrasound diagnosis method. Subsequently, for example, the guide wire 21 is percutaneously guided into a blood vessel in advance through a Seldinger's method, and the guide wire 21 is inserted through the wire lumen 16a of the inner tube 16 from the distal end opening portion 18a of the distal tip 18. While the guide wire 21 is guided out through the opening portion 22, the catheter 10A is inserted into a blood vessel. Under a radioscopic condition, the guide wire 21 is caused to advance toward a target lesion. The guide wire 21 is caused to pass through the lesion and to indwell, and the catheter 10A is caused to advance along the guide wire 21.

When the distal tip 18 of the catheter 10A passes through the lesion, the balloon 14 is positioned at the lesion. When the inflation fluid (for example, contrast agent) is pressure-fed into the dilation lumen 12a from the hub 20 side, the balloon 14 is inflated and the lesion is widened. Accordingly, treatment of the lesion can be performed. Subsequently, the inflation fluid is suctioned from the inside of the balloon 14 to the hub 20 side through the inflation lumen 12a, and the balloon 14 is deflated again. In a case where an additional lesion required to be treated is present at a different spot inside a body lumen, the balloon 14 is delivered to the additional lesion, the balloon 14 is inflated and deflated in a similar manner as described above. When the procedure for all of the lesion areas in a treatment object is completed, the catheter 10A is removed from the body.

In this case, as described above, in the reinforcement member 28 of the catheter 10A according to the present embodiment, at least one of the first end portion 31 and the second end portion 32, and the intermediate portion 34 are not fixed to the balloon 14. Here, the expression "at least one of the first end portion 31 and the second end portion 32, and the intermediate portion 34 are not directly fixed to the inner layer 24 and the outer layer 26" denotes that at least one of the first end portion 31 and the second end portion 32, and the intermediate portion 34 are not bonded to the inner layer 24 and the outer layer 26 and are not embedded in the inner layer 24 and the outer layer 26, thereby being able to freely move inside a space formed between the inner layer 24 and the outer layer 26. In other words, substantially the entirety of the reinforcement member 28 has the degree of freedom for moving in the axial direction and the circumferential direction with respect to the balloon 14. Therefore, favorable flexibility of the balloon 14 can be maintained. Accordingly, it is possible to realize the balloon 14 having high passing properties inside a body lumen.

Particularly, in a case of the present embodiment, not only one of the first end portion 31 and the second end portion 32 but also the other is not fixed to any one of the inner layer 24 and the outer layer 26. According to the configuration, the reinforcement member 28 is not fixed to any place in the balloon 14. Therefore, the degree of freedom for moving the reinforcement member 28 with respect to the balloon 14 can be further improved, and flexibility can be improved. In accordance therewith, crossability inside a body lumen can be further improved.

In addition, the reinforcement member 28 is disposed between the inner layer 24 and the outer layer 26 having elastic stretching properties. Therefore, high-pressure resistance and low compliance properties can be suitably applied to the balloon 14.

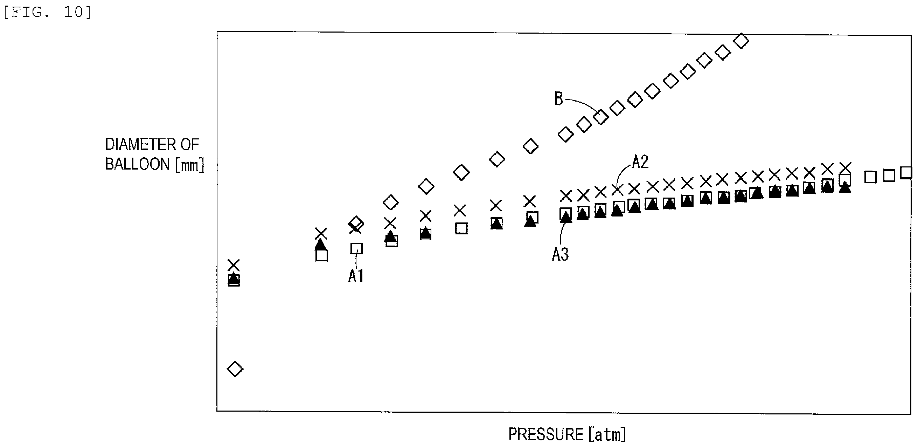

Here, Fig. 10 is a graph illustrating a relationship between pressure and a balloon diameter regarding balloons A1 to A3 which are provided with the reinforcement member 28 and in which forms of fixing the reinforcement member 28 are different from each other, and a balloon B provided with no reinforcement member 28. In Fig. 10, in the balloon A1, both the end portions of the reinforcement member 28 in the axial direction are fixed to the inner layer 24. In the balloon A2, only one end portion of the reinforcement member 28 in the axial direction is fixed to the inner layer 24. In the balloon A3, the reinforcement member 28 is not fixed to any place.

According to Fig. 10, it is found that in the balloons A1 to A3 provided with the reinforcement member 28, compared to the balloon B provided with no reinforcement member 28, the increases of the balloon diameters with respect to the increases of the pressures are gentle, while having high pressure resistance and low compliance properties. Meanwhile, in the balloons A1 to A3 provided with the reinforcement member 28, no meaningful difference based on the forms of fixing the reinforcement member 28 is recognized. Therefore, it is understood that a balloon having high-pressure resistance and low compliance properties can be realized by providing the reinforcement member 28 between the inner layer 24 and the outer layer 26, regardless of whether or not the reinforcement member 28 is fixed. Therefore, from the viewpoint of maintaining favorable flexibility of the balloon 14 and improving crossability of the catheter 10A inside a body lumen, it is favorable that at least one of both the end portions of the reinforcement member 28, and the intermediate portion 34 are not fixed to the inner layer 24 and the outer layer 26 of the balloon 14.

In addition, in a case of the present embodiment, the balloon 14 is inflated and deflated while entailing elastic stretching and is a zero folding-type balloon which is not folded when being in a deflated state. Accordingly, the balloon can easily restore the original outer diameter when being deflated again after inflation. Therefore, in a case where multiple lesion occurred in locations different from each other inside a body lumen are treated with the same balloon 14, the outer diameter after being deflated again is restrained from becoming greater than the initial outer diameter. Therefore, even after the balloon 14 is deflated again, favorable crossability inside a body lumen can be maintained.

Furthermore, the balloon 14 having elastic stretching properties can be prepared without performing blow molding. Therefore, the catheter 10A can be conveniently manufactured. In other words, in a case of a balloon which is configured with a non-stretchable material, the balloon is required to be molded so as to have a desired shape by executing blow molding after manufacturing the base material of the balloon. Moreover, in order to cause the balloon to be in a deflated state, there is a need to execute a wrapping step in which the balloon is folded (one or more outer circumferential portions of the balloon are folded in the circumferential direction in an overlapping manner) . In contrast, in a case of the balloon 14 of the present embodiment, as it is clear from the above-described manufacturing method, blow molding is not necessary and the wrapping step thereafter is also not necessary. Therefore, it is possible to reduce the number of steps and to lower the manufacturing cost.

In addition, in a case of the present embodiment, in the first end portion 31 and the second end portion 32 of the reinforcement member 28, inflation in the circumferential direction and the radial direction is restricted by the inflation restriction portion 36 (refer to Fig. 2). According to the configuration, in the reinforcement member 28, the maximally inflated diameter of the intermediate portion 34 positioned between the first end portion 31 and the second end portion 32 can be effectively restricted. Therefore, the function as the reinforcement member 28 can be suitably conducted.

In a case of the present embodiment, the reinforcement member 28 is formed by tubularly knitting one or more threads 29, and the waved threads 29 adjacent to each other in the axial direction are interlaced (refer to Fig. 3). According to the configuration, when the reinforcement member 28 is compressed in the circumferential direction, the threads 29 are folded in the circumferential direction, and when the reinforcement member 28 is compressed in the axial direction, the threads 29 of the meshes are misaligned in the axial direction. Therefore, the reinforcement member 28 can be flexibly bent.

In addition, in the reinforcement member 28 in which the meandering threads 29 adjacent to each other in the axial direction are interlaced with each other, the interlaced portion of the threads 29 configures an interlock portion. In the interlock portion, the threads 29 are not bonded to each other, and the threads 29 are formed so as to be movable with respect to each other. According to the configuration, the reinforcement member 28 can be bent in accordance with rotations of the interlaced portion of the threads 29. Therefore, flexibility of the balloon 14 can be further enhanced.

In a case of the present embodiment, the reinforcement member 28 is formed of high-strength fibers of which tensile break strength is equal to or greater than 2 GPa and an elastic modulus is equal to or greater than 50 GPa. According to the configuration, it is possible to realize the balloon 14 having excellent high-pressure resistance and low compliance properties.

### [Second Embodiment]

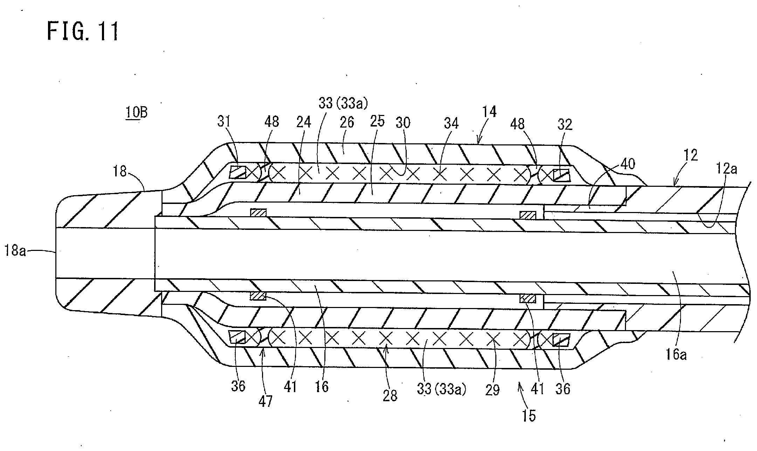

As illustrated in Fig. 11, in a catheter 10B according to the second embodiment of the present invention, a joint structure 47 that partially joins the inner layer 24 and the outer layer 26 to each other via a gap 33 penetrating the inner and outer surfaces of the reinforcement member 28 and that is not fixed to the reinforcement member 28 is provided such that the reinforcement member 28 is restrained from being misaligned in the axial direction from the initial position with respect to the inner layer 24 and the outer layer 26. In the present embodiment, the gap 33 is a gap 33 between the thread 29 and the thread 29 configuring the reinforcement member 28 (mesh 33a) .

The joint structure 47 has multiple welded portions 48 respectively penetrating multiple gaps 33 (meshes 33a). Each of the welded portions 48 is a portion formed after a part of the inner layer 24 and a part of the outer layer 26 are individually fused and solidified. The threads 29 configuring the reinforcement member 28, and the welded portions 48 are not welded to each other, that is, the threads 29 and the welded portions 48 are not fixed to each other. Therefore, the threads 29 are movable with respect to the welded portion 48.

In a case of the present embodiment, the joint structure 47 (welded portion 48) is provided in only a region of the reinforcement member 28 on the first end portion 31 side and a region of the reinforcement member 28 on the second end portion 32 side. In other words, no joint structure 47 is provided between the region on the first end portion 31 side and the region on the second end portion 32 side. The region of the reinforcement member 28 on the first end portion 31 side can include not only the first end portion 31 and a part in the vicinity thereof but also, for example, a region to the extent of 1/3 to 1/4 the overall length of the reinforcement member 28 from the outermost distal end position of the reinforcement member 28. In addition, the region of the reinforcement member 28 on the second end portion 32 side can include not only the second end portion 32 and a part in the vicinity thereof but also a region to the extent of 1/3 to 1/4 the overall length of the reinforcement member 28 from the innermost proximal end position of the reinforcement member 28.

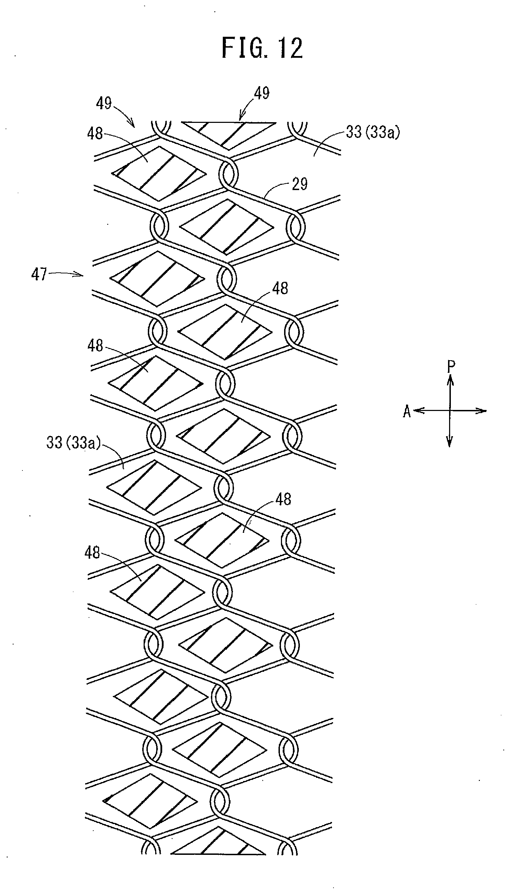

Fig. 12 is a schematic view in which the reinforcement member 28 and the joint structure 47 (joint structure 47 on the first end portion 31 side) are developed in the circumferential direction. In Fig. 12, an arrow A direction is the axial direction, and an arrow P direction is the circumferential direction. In the present embodiment, the welded portions 48 are provided in each of the meshes 33a adjacent to each other in the circumferential direction so as to be spaced from each other in the circumferential direction, and a welded portion row 49 is configured with the multiple welded portions 48 which are arranged in the circumferential direction. In addition, multiple welded portion rows 49 are provided in the axial direction of the reinforcement member 28, and the welded portions 48 are misaligned in the circumferential direction between the welded portion rows 49. Note that, the joint structure 47 on the second end portion 32 side is also configured in a manner similar to the joint structure 47 on the first end portion 31 side.

As illustrated in Fig. 12, the thread 29 is not fixed to the welded portion 48, and the space between the welded portions 48 adjacent to each other has a size equal to or greater than the thickness of the thread 29. Therefore, the thread 29 is movable with respect to the welded portion 48. Meanwhile, the welded portion 48 is disposed through the gap 33 (mesh 33a) of the reinforcement member 28. Therefore, when the reinforcement member 28 moves, the thread 29 is caught by the welded portion 48, and thus, the reinforcement member 28 is restricted from moving in the axial direction.

Accordingly, the reinforcement member 28 has the appropriate degree of freedom for moving between the inner layer 24 and the outer layer 26. However, the reinforcement member 28 is not significantly misaligned in the axial direction from the initial position. Therefore, the innermost proximal end position of the reinforcement member 28 is held on the proximal side beyond the stretchable region 25 of the inner layer 24, and thus, the reinforcement member 28 is prevented from being misaligned toward the distal side beyond the innermost proximal end position of the stretchable region 25.

In the reinforcement member 28, the gap 33 which the welded portion 48 penetrates is not limited to the mesh 33a. The gap 33 may be a space other than the mesh 33a. For example, the gap 33 in a form of a hole or a slit penetrating the inner and outer surfaces may be formed in each of the inflation restriction portions 36 on both sides of the reinforcement member 28 in the axial direction, and the welded portion 48 may be provided so as to penetrate the gap 33. In this case, in each of the inflation restriction portions 36, it is favorable that multiple gaps 33 in the forms of holes or slits are formed so as to be spaced from each other in the circumferential direction and the welded portion 48 penetrates each of the gaps 33.

Note that, the configurations other than the distal portion of the catheter 10B not illustrated in Fig. 11 are configured in a manner similar to the catheter 10A illustrated in Fig. 1.

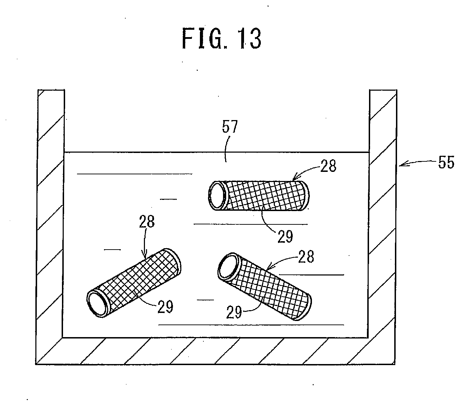

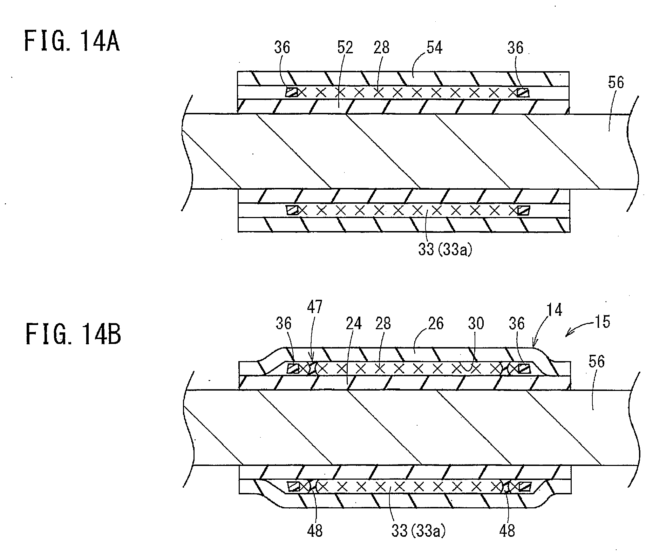

Subsequently, an example of a method of manufacturing the catheter 10B (mainly, the step of manufacturing the dilation portion 15 and peripheral portions thereof) will be described. Note that, the present invention is not limited to the exemplified manufacturing method. In Figs. 13, 14A, and 14B, the tubularly net-shaped reinforcement member 28 is schematically illustrated, and it is not intended to form the reinforcement member 28 through a particular knitting method.

First, similar to the method of manufacturing the catheter 10A, the step of preparing a base material sleeve (Fig. 4A), and the cutting and restriction processing step (Fig. 4B) are executed, and the multiple reinforcement members 28 are obtained.

Subsequently, a step of wetting the reinforcement member 28 with liquid 57 (wetting step) is executed. The liquid 57 applied in this step is used for inhibiting the thread 29 configuring the reinforcement member 28, and the inner layer 24 and the outer layer 26 from being welded to each other in a welding step to be described below. Examples of the liquid 57 include water and oil (silicone oil, and the like).

In the wetting step, for example, as illustrated in Fig. 13, the reinforcement member 28 may be wetted with the liquid 57 by dipping the reinforcement member 28 in the liquid 57 which is input into a container 55, and taking out the reinforcement member 28 from the liquid 57, thereafter. The thread 29 configuring the reinforcement member 28 is a twisted thread which is formed by twisting fibers . Therefore, when the liquid 57 enters between the fibers, the thread 29 is wetted. As another method, the reinforcement member 28 may be wetted with the liquid 57 by applying (pouring) the liquid 57, spraying the liquid 57, painting the thread 29 with a brush, and the like. Note that, the reinforcement member 28 wetted with the liquid 57 may be obtained by knitting the threads 29 which are wetted with the liquid 57 in advance.

Subsequently, a disposition step of disposing the reinforcement member 28 between the inner layer 24 and the outer layer 26 is executed. For example, the disposition step includes the first covering step and the second covering step described below.

In the first covering step, similar to the method of manufacturing the catheter 10A, the inner layer tube 52 which is the base material of the inner layer 24 is covered with the reinforcement member 28 wetted with the liquid 57 (refer to Fig. 5A). Note that, the reinforcement member 28 may be wetted with the liquid 57 after the inner layer tube 52 is covered with the reinforcement member 28 which is not wetted with the liquid 57. Subsequently, in the second covering step, similar to the method of manufacturing the catheter 10A, the inner layer tube 52 and the reinforcement member 28 are covered with the outer layer tube 54 (refer to Fig. 5B).

Subsequently, similar to the method of manufacturing the catheter 10A, the step of joining the inner and outer layers (refer to Figs. 6A and 6B) is executed such that the annularly sealed accommodation chamber 30 is formed between the inner layer 24 and the outer layer 26.

Subsequently, as illustrated in Figs. 14A and 14B, the welding step of partially welding the inner layer 24 and the outer layer 26 to each other via the gap 33 (mesh 33a) penetrating the inner and outer surfaces of the reinforcement member 28 is executed without fixing the reinforcement member 28 to the inner layer 24 and the outer layer 26. In this case, without heating the entirety of the balloon 14, portions corresponding to the first end portion 31 side and the second end portion 32 side of the reinforcement member 28 are heated by being irradiated with a laser or the like, and thus, the inner layer 24 and the outer layer 26 can be welded to each other at only the heated portions. Accordingly, the welded portion 48 can be formed in only the region on the first end portion 31 side and the region on the second end portion 32 side of the reinforcement member 28.

In the welding step, the reinforcement member 28 wetted with the liquid 57 (refer to Fig. 13) is not welded to the inner layer 24 and the outer layer 26. In other words, since the surface of the reinforcement member 28 is wetted with the liquid 57, even if the reinforcement member 28 is in contact with the inner layer 24 and the outer layer 26, the materials (resin) of the inner layer 24 and the outer layer 26 do not reach the fusing point or more at the contact portions. As a result, the reinforcement member 28, and the inner layer 24 and the outer layer 26 are inhibited from being welded to each other.

Note that, in the welding step, the welded portion 48 may be formed by irradiating only the spots of the meshes 33a in the reinforcement member 28 with a laser such that the inner layer 24 and the outer layer 26 are welded to each other. In this case, even though the reinforcement member 28 is not wetted with the liquid 57, the inner layer 24 and the outer layer 26 (positions in the inner layer 24 and the outer layer 26 avoiding the threads 29) can be welded to each other by selecting only the spots corresponding to the meshes 33a. In addition, when the outer layer 26 is configured with a material having transparent properties, positions of the meshes 33a in the reinforcement member 28 disposed on the inner side of the outer layer 26 can be easily checked.

In this manner, it is possible to obtain the inflation portion 15 in a state where the reinforcement member 28 is movably disposed inside the accommodation chamber 30 of the balloon 14 within a restricted range. After the welding step, the mandrel 56 is removed.

In this case, in the present embodiment, the reinforcement member 28 is merely disposed inside the accommodation chamber 30 and is not joined to other members through welding, bonding, or the like. Therefore, the reinforcement member 28 is not fixed to any portion of the balloon 14 (inner layer 24 and outer layer 26).

Subsequently, similar to the method of manufacturing the catheter 10A, the step of joining a balloon and a shaft (refer to Figs. 7A and 7B), a step of fixing the radiopaque marker 41 to an inner tube (not illustrated), the step of joining a balloon and an inner tube (refer to Figs. 8A and 8B), and the step of joining a distal tip and an inner tube (refer to Figs. 9A and 9B) are sequentially executed. Note that, in the method of manufacturing the catheter 10B, steps other than those mentioned above are similar to the method of manufacturing the catheter 10A according to the first embodiment.

The catheter 10B configured as described above can perform treatment of a lesion occurring inside a body lumen through a using method similar to the catheter 10A according to the first embodiment.

In this case, in the catheter 10B according to the present embodiment, high-pressure resistance and low compliance properties can be applied to the balloon 14 due to the reinforcement member 28, and the reinforcement member 28 has the degree of freedom for moving with respect to the balloon 14. Therefore, favorable flexibility of the balloon 14 can be maintained. Accordingly, it is possible to realize the balloon 14 having high crossability inside a body lumen.

In addition, in the catheter 10B, the joint structure 47 that partially joins the inner layer 24 and the outer layer 26 to each other via the gap 33 penetrating the inner and outer surfaces of the reinforcement member 28 and that is not fixed to the thread 29 (wire member) is provided such that the reinforcement member 28 is appropriately restrained from being misaligned in the axial direction from the initial position. Therefore, the reinforcement member 28 is not significantly misaligned in the axial direction, and unreinforced portions of the inner layer 24 and the outer layer 26 are not exposed. Accordingly, a state of the balloon 14 reinforced by the reinforcement member 28 can be suitably maintained. Therefore, even after inflation and deflation of the balloon 14 are repeated multiple times, the balloon 14 is not ruptured while being under low pressure, and thus, the catheter 10B can be stably used.

The joint structure 47 has one or more welded portions 48 penetrating the gaps 33 in the reinforcement member 28, and the wire member is movable with respect to the welded portion 48. According to the configuration, the thread 29 is caught by the welded portion 48. Therefore, the reinforcement member 28 is appropriately restrained from being misaligned. Therefore, it is possible to simply realize a structure in which the reinforcement member 28 is effectively restrained from being misaligned in the axial direction, without fixing the reinforcement member 28 to the inner layer 24 and the outer layer 26 through the joint structure 47.

In a case of the present embodiment, the joint structure 47 is provided in only the region of the reinforcement member 28 on the first end portion 31 side and the region of the reinforcement member 28 on the second end portion 32 side. According to the configuration, while achieving favorable flexibility of the balloon, the reinforcement member 28 can be effectively restrained from being misaligned in the axial direction.

In a case of the present embodiment, the gaps 33 are the meshes 33a in the reinforcement member 28. According to the configuration, the meshes 33a in the reinforcement member 28 can be utilized. Therefore, there is no need to provide a dedicated hole or the like for joining the inner layer 24 and the outer layer 26 to each other, in the reinforcement member 28.

In addition, according to the catheter manufacturing method of the present example, the balloon 14 has flexibility as well as high-pressure resistance and low compliance properties, and the reinforcement member 28 is restrained from being misaligned in the axial direction with respect to the balloon 14. Therefore, it is possible to manufacture the catheter 10B including the balloon 14 which is not ruptured while being under low pressure even after inflation and deflation are repeated multiple times.

In a case of the present example, in the welding step, in a state where the reinforcement member 28 wetted with the liquid 57 is disposed between the inner layer 24 and the outer layer 26, the inner layer 24 and the outer layer 26 are heated and are welded to each other. Accordingly, in the welding step, the reinforcement member 28 wetted with the liquid 57 is not welded to the inner layer 24 and the outer layer 26. Thus, it is possible to simply form a structure in which even though the inner layer 24 and the outer layer 26 are partially welded to each other, the reinforcement member 28 is movable with respect to the inner layer 24 and the outer layer 26.

In addition, in a case of the present embodiment, in the welding step, one or more regions in each of the inner layer 24 and the outer layer 26 in the axial direction are selectively heated. Therefore, while achieving favorable flexibility of the balloon 14, the reinforcement member 28 can be effectively restrained from being misaligned in the axial direction. In this case, in the welding step, only a spot of the gap 33 in the reinforcement member 28 is irradiated with a laser such that the inner layer 24 and the outer layer 26 are welded to each other. Therefore, without wetting the reinforcement member 28 with the liquid 57, the inner layer 24 and the outer layer 26 can be welded to each other at a spot avoiding the wire member.

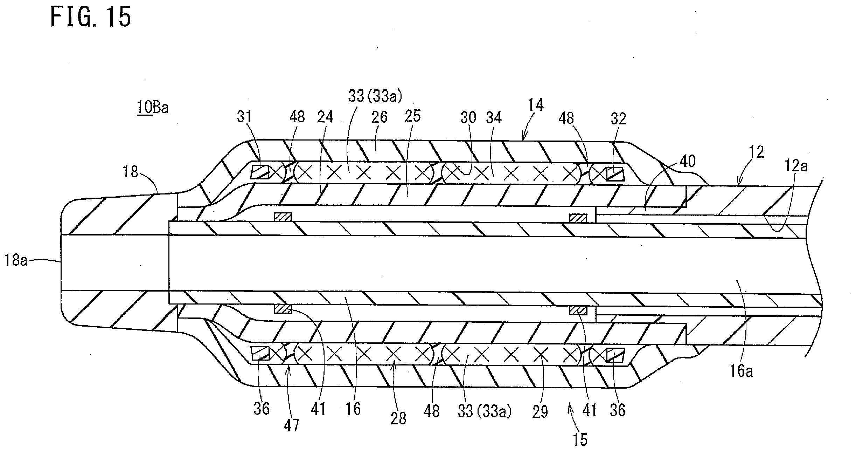

Note that, as in a catheter 10Ba illustrated in Fig. 15, the welded portion 48 may be provided in the region on the first end portion 31 side, the region on the second end portion 32 side, and a central portion of the reinforcement member 28 in the axial direction.

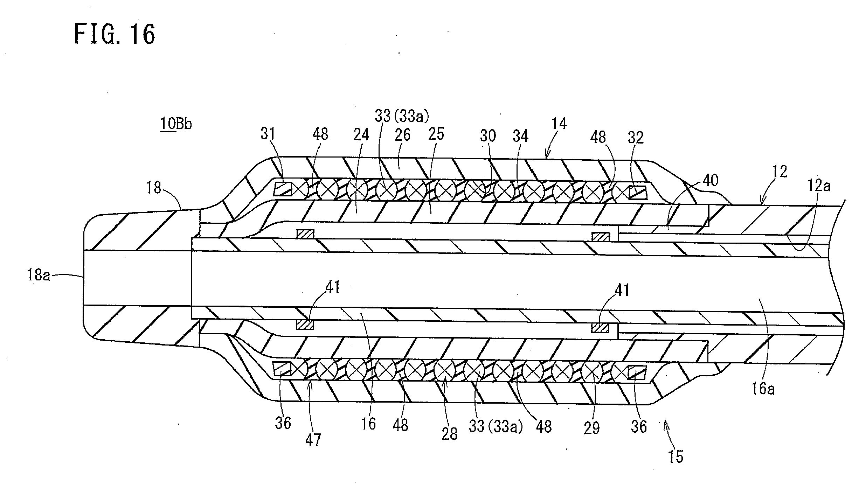

As in a catheter 10Bb illustrated in Fig. 16, the multiple welded portions 48 configuring the joint structure 47 may be disposed in a dispersive manner while being spaced from each other, throughout substantially the entirety between the inner layer 24 and the outer layer 26 (substantially the entirety of the reinforcement member 28). In a case of manufacturing the catheter 10Bb, in the above-described welding step, the entirety of the balloon 14 in a state where the reinforcement member 28 wetted with the liquid 57 is disposed between the inner layer 24 and the outer layer 26 may be heated. Accordingly, the welding step of welding the inner layer 24 and the outer layer 26 to each other via the gap 33 can be efficiently carried out. Note that, in the welding step, instead of heating the entirety of the balloon 14, the inner layer 24 and the outer layer 26 may be welded to each other via the gap 33 by irradiating only a spot of the gap 33 in the reinforcement member 28 of the balloon 14 with a laser.

Note that, in the second embodiment, regarding the common portions of the first embodiment, the operations and the effects which are the same as or similar to those of the first embodiment can be obtained.

### [Third Embodiment]

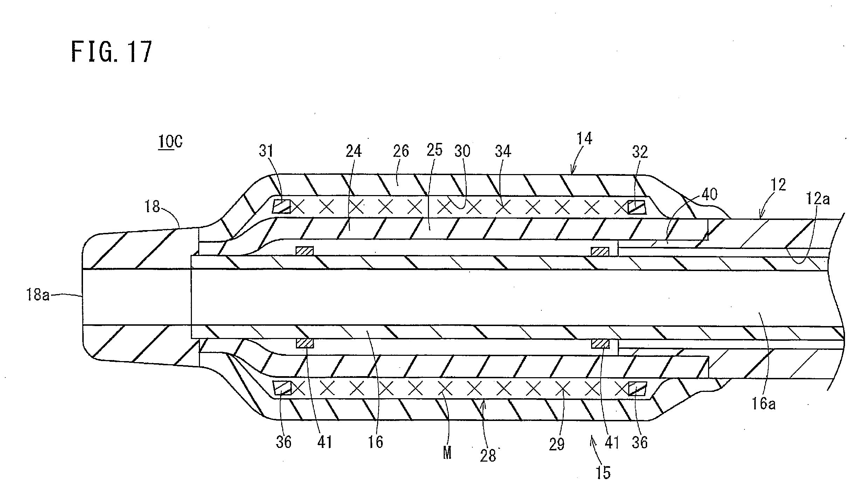

A catheter 10C according to the third embodiment illustrated in Fig. 17 is different from the catheter 10A according to the above-described first embodiment in that a lubricant M is present on at least the outer surface of the thread 29. Specifically, in the balloon 14 of the catheter 10C, the lubricant M is present on at least the outer surface of the thread 29 such that friction between portions of the threads 29 in contact with each other (portions interlaced with each other) is reduced. In other words, due to the lubricant M, movement of portions of the threads 29 in contact with each other becomes lubricative with each other.

In the present embodiment, the lubricant M is applied to the thread 29. The lubricant M may be any one of a liquid lubricant, a semisolid lubricant (grease), and a solid lubricant. Examples of the liquid lubricant include mineral oil, synthetic oil (silicone oil, and the like), and fatty oil. Examples of the solid lubricant include polytetrafluoroethylene. Note that, the lubricant is a substance which restrains friction or abrasion acting between two substances.

In a case where the thread 29 is a twisted thread and the lubricant M is a liquid lubricant, the lubricant M is present not only on the outer surface of the thread 29 but also the lubricant M is in a state of entering between fibers configuring the thread 29, that is, the lubricant M is in a state of being impregnated in the thread 29.

Note that, the configurations other than the distal portion of the catheter 10C not illustrated in Fig. 17 are configured in a manner similar to the catheter 10A illustrated in Fig. 1.

Subsequently, an example of a method of manufacturing the catheter 10C (mainly, the step of manufacturing the dilation portion 15 and peripheral portions thereof) will be described.

The method of manufacturing the catheter 10C further includes a step of applying the lubricant M to the thread 29 (fibers) configuring the reinforcement member 28 (applying step), compared to the method of manufacturing the catheter 10A according to the first embodiment (refer to Figs. 4A to 9B). This applying step is executed between the cutting and restriction processing step (refer to Fig. 4B) of forming the inflation restriction portion 36 at both the end portions of the divided sleeve 51, and the first covering step (refer to Fig. 5A) covering the inner layer tube 52 with the reinforcement member 28.

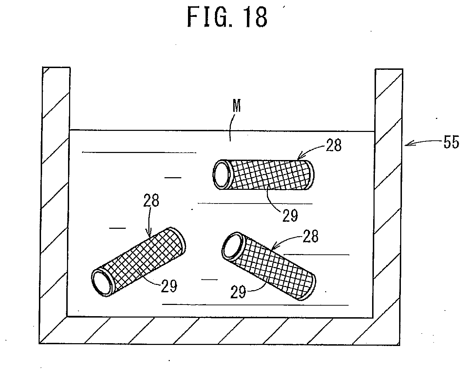

In a case where the lubricant M is liquid, in the applying step, for example, as illustrated in Fig. 18, the lubricant M may be applied to the reinforcement member 28 by dipping the reinforcement member 28 in the lubricant M which is input into the container 55, and taking out the reinforcement member 28. Since the thread 29 configuring the reinforcement member 28 is a twisted thread which is formed by twisting fibers. Therefore, when the lubricant M enters between the fibers, the lubricant M is impregnated in the thread 29. As another method, the lubricant M may be applied to the reinforcement member 28 by applying (pouring) the lubricant M, spraying the lubricant M, painting the reinforcement member 28 with a brush, and the like. Note that, the reinforcement member 28 in which the lubricant M is applied to the thread may be obtained by knitting the thread 29 to which the lubricant M is applied in advance.

The catheter 10C configured as described above can perform treatment of a lesion occurring inside a body lumen through a using method similar to the catheter 10A according to the first embodiment.

In this case, in the catheter 10C according to the present embodiment, the lubricant M is present on at least the outer surface of the thread 29 (wire member) such that friction between the threads 29 (portions of the threads 29 in contact with each other) is reduced. According to the configuration, since the lubricant M makes movement of the threads 29 lubricative with each other, friction between the threads 29 can be reduced, and the degree of freedom of movement between the threads 29 can be enhanced. Thus, when being deflated again after inflation, the balloon 14 can easily restore the original shape (the thickness) before being inflated.

In a case of the present embodiment, since the lubricant M is applied to the threads 29, the amount of the using lubricant M can be restrained, and friction between the portions of the thread 29 in contact with each other can be efficiently reduced.

In a case of the present embodiment, the reinforcement member 28 is formed of high-strength fibers of which tensile break strength is equal to or greater than 2 GPa and an elastic modulus is equal to or greater than 50 GPa. According to the configuration, it is possible to realize the balloon 14 having excellent high-pressure resistance and low compliance properties.

In addition, generally, frictional resistance between high-strength fibers (super fibers) is high. Therefore, in a case where the reinforcement member 28 is configured with the high-strength fiber, if the fibers are in direct contact with each other without being lubricated, there is a possibility that when the balloon 14 is deflated again, it may be difficult for the balloon 14 to restore the original shape before being inflated. In contrast, in the present embodiment, when the configuration in which the lubricant M is present on the outer surface of the thread 29 formed of the high-strength fiber is employed, a problem of frictional resistance occurring when a high-strength fiber is used as the thread 29 can be restrained, and high-pressure resistance and low compliance properties can be effectively applied to the balloon 14.

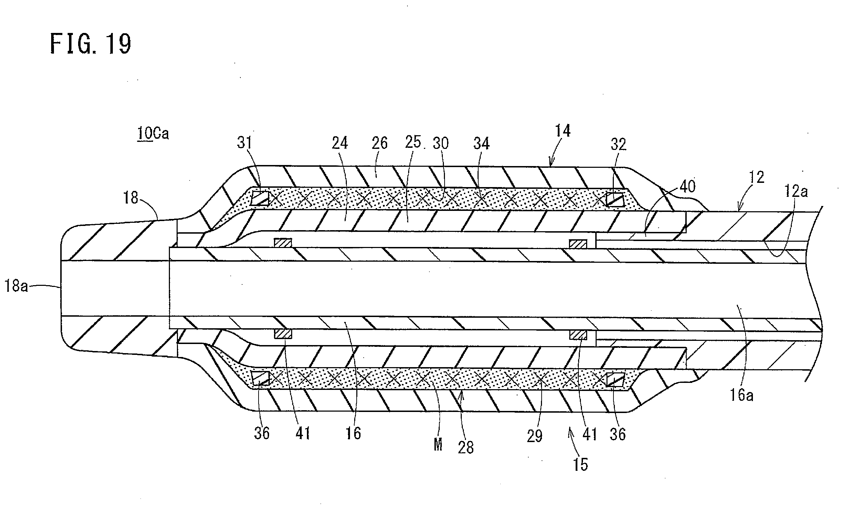

Fig. 19 is a schematic cross-sectional view of the distal portion of a catheter 10Ca according to a modification example of the third embodiment. In the catheter 10Ca, the accommodation chamber 30 formed between the inner layer 24 and the outer layer 26 is filled with the lubricant M. In this case, the lubricant M is in a liquefied state or a semisolid state. According to the configuration, a state where the lubricant M is present around the thread 29 configuring the reinforcement member 28 can be maintained. Therefore, friction between the portions of the threads 29 in contact with each other can be reliably reduced. Note that, since the accommodation chamber 30 is a sealed space, the lubricant M does not leak out from the balloon 14.

In the third embodiment, regarding the common portions of the first embodiment, the operations and the effects which are the same as or similar to those of the first embodiment can be obtained.

The present invention is not limited to the embodiments described above, and various modifications and changes can be made without departing from the scope of the present invention.

## Claims

1. A catheter (10A, 10B, 10Ba, 10Bb, 10C, 10Ca] comprising:
a balloon (14) that has an inner layer (24) and an outer layer (26) having elastic stretching properties, having tubular shapes, and being able to be inflated and deflated in response to a change of internal pressure; and
a tubularly net-shaped reinforcement member (28) that is disposed between the inner layer (24) and the outer layer (26),
wherein the reinforcement member (28) has a first end portion (31) and a second end portion (32) in an axial direction,
and an intermediate portion (34) configuring a portion between the first end portion (31) and the second end portion (32),
and
**characterized in that** at least one of the first end portion (31) and the second end portion (32), and the intermediate portion (34) are not directly fixed to the inner layer (24) and the outer layer (26).

2. The catheter (10A, 10B, 10Ba, 10Bb, 10C, 10Ca) according to Claim 1,
wherein the other of the first end portion (31) and the second end portion (32) is not also directly fixed to the inner layer (24) and the outer layer (26).

3. The catheter (10A, 10B, 10Ba, 10Bb, 10C, 10Ca) according to Claim 1 or 2,
wherein inflation of the first end portion (31) and the second end portion (32) in a circumferential direction is restricted.

4. The catheter [10A, 10B, 10Ba, 10Bb, 10C, 10Ca) according to any one of Claims 1 to 3,
wherein the reinforcement member (28) is formed by tubularly knitting one or more threads (29), and the waved threads (29) adjacent to each other in the axial direction are interlaced with each other.

5. The catheter [10A, 10B, 10Ba, 10Bb, 10C, 10Ca) according to any one of Claims 1 to 4,
wherein the reinforcement member (28) is formed of a high-strength fiber of which tensile break strength is equal to or greater than 2 GPa and an elastic modulus is equal to or greater than 50 GPa.

6. The catheter (10A, 10B, 10Ba, 10Bb, 10C, 10Ca) according to any one of Claims I to 5,
wherein an axial-directional length of the reinforcement member (28) is greater than an axial-directional length of a region (25) in the inner layer (24) that is stretchable during deformation of inflation and deflation of the balloon (14), and
wherein an axial-directional length of the outer layer (26) is greater than the axial-directional length of the reinforcement member (28).

7. The catheter (10B, 10Ba, 10Bb) according to Claim 1,
wherein a joint structure (47) that partially joins the inner layer (24) and the outer layer (26) to each other via multiple gaps (33) penetrating inner and outer surfaces of the reinforcement member (28) and that is not fixed to the reinforcement member (28) is provided such that the reinforcement member (28) is restrained from being misaligned in the axial direction with respect to the balloon (14).

8. The catheter (10B, 10Ba, 10Bb) according to Claim 7,
wherein the joint structure (47) has multiple welded portions (48) respectively penetrating the multiple gaps (33),
and
wherein the reinforcement member (28) is movable with respect to the welded portions (48).

9. The catheter (10B) according to Claim 7 or 8,
wherein the joint structure (47) is provided in only a region of the reinforcement member (28) on the first end portion (31) side and a region of the reinforcement member (28) on the second end portion (32) side.

10. The catheter (10B, 10Ba, 10Bb) according to any one ofClaims 7 to 9,
wherein the multiple gaps (33) are meshes (33a) in the reinforcement member (28).

11. The catheter (10e, 10Ca) according to Claim 1,
wherein the reinforcement member (28) is formed so as to include one or more wire members (29), and
wherein a lubricant (M) is present on at least an outer surface of the wire member (29) such that friction between portions of the wire members(29) in contact with each other is reduced.

12. The catheter (10C) according to Claim 11,
wherein the lubricant (M) is applied to the wire member (29).

13. The catheter (10Ca) according to Claim 11,
wherein an accommodation chamber (30) accommodating the reinforcement member (28) is formed between the inner layer (24) and the outer layer (26), and
wherein the accommodation chamber (30) is filled with the lubricant (M).

14. The catheter (10C, 10Ca) according to any one of Claims 11 to 13,
wherein the wire member (29) is formed of a high-strength fiber of which tensile break strength is equal to or greater than 2 GPa and an elastic modulus is equal to or greater than 50 GPa.

## Patentansprüche

1. Katheter (10A, 10B, 10Ba, 10Bb, 10C, 10Ca), der Folgendes umfasst:
einen Ballon (14), der eine innere Lage (24) und eine äußere Lage (26) mit elastischen Dehneigenschaften besitzt, der eine schlauchartige Gestalt besitzt und in Reaktion auf eine Änderung des Innendrucks aufgeblasen und entleert werden kann; und
ein Verstärkungselement (28) in Form eines schlauchartigen Netzes, das zwischen der inneren Lage (24) und der äußeren Lage (26) angeordnet ist,
wobei das Verstärkungselement (28) einen ersten Endabschnitt (31) und einen zweiten Endabschnitt (32) in einer axialen Richtung besitzt,
und einen mittleren Abschnitt (34), der einen Abschnitt zwischen dem ersten Endabschnitt (31) und dem zweiten Endabschnitt (32) bildet,
und
**dadurch gekennzeichnet, dass** mindestens einer von dem ersten Endabschnitt (31) und dem zweiten Endabschnitt (32) sowie der mittlere Abschnitt (34) nicht direkt an der inneren Lage (24) und der äußeren Lage (26) befestigt sind.

2. Katheter (10A, 10B, 10Ba, 10Bb, 10C, 10Ca) nach Anspruch 1,
wobei der andere von dem ersten Endabschnitt (31) und dem zweiten Endabschnitt (32) nicht ebenfalls direkt an der inneren Lage (24) und der äußeren Lage (26) befestigt ist.

3. Katheter (10A, 10B, 10Ba, 10Bb, 10C, 10Ca) nach Anspruch 1 oder 2,
wobei das Aufblasen des ersten Endabschnitts (31) und des zweiten Endabschnitts (32) in einer Umfangsrichtung eingeschränkt ist.

4. Katheter (10A, 10B, 10Ba, 10Bb, 10C, 10Ca) nach einem der Ansprüche 1 bis 3,
wobei das Verstärkungselement (28) durch schlauchartiges Stricken eines oder mehrerer Fäden (29) gebildet ist und die in axialer Richtung aneinander angrenzenden gewellten Fäden (29) miteinander verflochten sind.

5. Katheter (10A, 10B, 10Ba, 10Bb, 10C, 10Ca) nach einem der Ansprüche 1 bis 4, wobei das Verstärkungselement (28) aus einer hochfesten Faser gebildet ist, deren Reißfestigkeit gleich oder größer ist als 2 GPa und deren Elastizitätsmodul gleich oder größer ist als 50 GPa.

6. Katheter (10A, 10B, 10Ba, 10Bb, 10C, 10Ca) nach einem der Ansprüche 1 bis 5, wobei eine in axialer Richtung verlaufende Länge des Verstärkungselements (28) größer ist als eine in axialer Richtung verlaufende Länge eines Bereichs (25) in der inneren Lage (24), der während der Verformung beim Aufblasen und Entleeren des Ballons (14) dehnbar ist, und
wobei eine Länge der äußeren Lage (26) in axialer Richtung größer ist als die Länge des Verstärkungselements (28) in axialer Richtung.

7. Katheter (10B, 10Ba, 10Bb) nach Anspruch 1,
wobei eine Verbindungsstruktur (47), die die innere Lage (24) und die äußere Lage (26) über eine Vielzahl von Öffnungen (33), die die Innen- und Außenseite des Verstärkungselements (28) durchdringen, teilweise miteinander verbindet und die nicht an dem Verstärkungselement (28) befestigt ist, so vorgesehen ist, dass das Verstärkungselement (28) nicht in axialer Richtung in Bezug auf den Ballon (14) verrutschen kann.

8. Katheter (10B, 10Ba, 10Bb) nach Anspruch 7,
wobei die Verbindungsstruktur (47) eine Vielzahl von geschweißten Abschnitten (48) besitzt, die jeweils die Vielzahl von Öffnungen (33) durchdringen,
und
wobei das Verstärkungselement (28) in Bezug auf die geschweißten Abschnitte (48) bewegbar ist.

9. Katheter (10B) nach Anspruch 7 oder 8,
wobei die Verbindungsstruktur (47) nur in einem Bereich des Verstärkungselements (28) auf der Seite des ersten Endabschnitts (31) und in einem Bereich des Verstärkungselements (28) auf der Seite des zweiten Endabschnitts (32) vorgesehen ist.

10. Katheter (10B, 10Ba, 10Bb) nach einem der Ansprüche 7 bis 9,
wobei die Vielzahl von Öffnungen (33) Maschen (33a) in dem Verstärkungselement (28) sind.

11. Katheter (10e, 10Ca) nach Anspruch 1,
wobei das Verstärkungselement (28) so ausgebildet ist, dass es ein oder mehrere Drahtelemente (29) aufweist, und
wobei ein Gleitmittel (M) wenigstens auf einer Außenseite des Drahtelements (29) vorhanden ist, so dass die Reibung zwischen Abschnitten der miteinander in Kontakt stehenden Drahtelemente (29) verringert wird.

12. Katheter (10C) nach Anspruch 11,
wobei das Gleitmittel (M) auf das Drahtelement (29) aufgebracht ist.

13. Katheter (10Ca) nach Anspruch 11,
wobei eine Aufnahmekammer (30), die das Verstärkungselement (28) aufnimmt, zwischen der inneren Lage (24) und der äußeren Lage (26) gebildet ist, und wobei die Aufnahmekammer (30) mit dem Gleitmittel (M) gefüllt ist.

14. Katheter (10C, 10Ca) nach einem der Ansprüche 11 bis 13,
wobei das Drahtelement (29) aus einer hochfesten Faser gebildet ist, deren Reißfestigkeit gleich oder größer ist als 2 GPa und deren Elastizitätsmodul gleich oder größer ist als 50 GPa.

## Revendications

1. Cathéter (10A, 10B, 10Ba, 10Bb, 10C, 10Ca) comprenant :
un ballonnet (14) qui comprend une couche intérieure (24) et une couche extérieure (26) ayant des propriétés d'étirement élastique, ayant des formes tubulaires, et pouvant être gonflé et dégonflé en réponse à un changement de pression interne ; et
un élément de renforcement en forme de filet tubulaire (28) qui est disposé entre la couche intérieure (24) et la couche extérieure (26),
dans lequel l'élément de renforcement (28) comprend une première partie terminale (31) et une seconde partie terminale (32) dans une direction axiale,
et une partie intermédiaire (34) qui constitue une partie entre la première partie terminale (31) et la seconde partie terminale (32),
et
**caractérisé en ce qu'**au moins l'une de la première partie terminale (31) et de la seconde partie terminale (32), et la partie intermédiaire (34) ne sont pas fixées directement à la couche intérieure (24) et à la couche extérieure (26).

2. Cathéter (10A, 10B, 10Ba, 10Bb, 10C, 10Ca) selon la revendication 1, dans lequel l'autre de la première partie terminale (31) et de la seconde partie terminale (32) n'est également pas fixée directement à la couche intérieure (24) et à la couche extérieure (26).

3. Cathéter (10A, 10B, 10Ba, 10Bb, 10C, 10Ca) selon la revendication 1 ou 2,
dans lequel le gonflement de la première partie terminale (31) et de la seconde partie terminale (32) dans une direction circonférentielle est limité.

4. Cathéter (10A, 10B, 10Ba, 10Bb, 10C, 10Ca) selon l'une quelconque des revendications 1 à 3,
dans lequel l'élément de renforcement (28) est formé en tricotant de façon tubulaire un ou plusieurs fils (29), et les fils ondulés (29) adjacents les uns aux autres dans la direction axiale sont entrelacés les uns avec les autres.

5. Cathéter (10A, 10B, 10Ba, 10Bb, 10C, 10Ca) selon l'une quelconque des revendications 1 à 4,
dans lequel l'élément de renforcement (28) est formé d'une fibre à haute résistance dont la résistance à la rupture par traction est supérieure ou égale à 2GPa et un module d'élasticité est supérieure ou égal à 50 GPa.

6. Cathéter (10A, 10B, 10Ba, 10Bb, 10C, 10Ca) selon l'une quelconque des revendications 1 à 5,
dans lequel une longueur dans la direction axiale de l'élément de renforcement (28) est supérieure à une longueur dans la direction axiale d'une région (25) de la couche intérieure (24) qui est étirable lors de la déformation se produisant au gonflement et au dégonflement du ballonnet (14), et
dans lequel une longueur dans la direction axiale de la couche extérieure (26) est supérieure à la longueur dans la direction axiale de l'élément de renforcement (28).

7. Cathéter (10B, 10Ba, 10Bb) selon la revendication 1,
dans lequel une structure de jonction (47) qui relie partiellement l'une à l'autre la couche intérieure (24) et la couche extérieure (26) via de multiples espaces (33) pénétrant dans les surfaces intérieure et extérieure de l'élément de renforcement (28) et qui n'est pas fixée à l'élément de renforcement (28) est prévue de telle sorte que l'élément de renforcement (28) ne peut pas être désaxé dans la direction axiale par rapport au ballonnet (14).

8. Cathéter (10B, 10Ba, 10Bb) selon la revendication 7,
dans lequel la structure de jonction (47) comprend de multiples parties soudées (48) qui pénètrent respectivement dans les multiples espaces (33),
et
dans lequel l'élément de renforcement (28) est mobile par rapport aux parties soudées (48).

9. Cathéter (10B) selon la revendication 7 ou 8,
dans lequel la structure de jonction (47) n'est prévue que dans une région de l'élément de renforcement (28) du côté de la première partie terminale (31) et dans une région de l'élément de renforcement (28) du côté de la seconde extrémité terminale (32).

10. Cathéter (10B, 10Ba, 10Bb) selon l'une quelconque des revendications 7 à 9,
dans lequel les multiples espaces (33) sont des mailles (33a) dans l'élément de renforcement (28).

11. Cathéter (10e, 10Ca) selon la revendication 1,
dans lequel l'élément de renforcement (28) est formé de manière à comprendre un ou plusieurs éléments de fil (29), et
dans lequel un lubrifiant (M) est présent sur au moins une surface extérieure de l'élément de fil (29) de telle sorte que le frottement entre des parties des éléments de fil (29) en contact les unes avec les autres est réduit.

12. Cathéter (10C) selon la revendication 11,
dans lequel 1 lubrifiant (M) est appliqué sur l'élément de fil (29).

13. Cathéter (10Ca) selon la revendication 11,
dans lequel une chambre de réception (30) logeant l'élément de renforcement (28) est formée entre la couche intérieure (24) et la couche extérieure (26), et
dans lequel la chambre de réception (30) est remplie du lubrifiant (M).

14. Cathéter (10C, 10Ca) selon l'une quelconque des revendications 11 à 13,
dans lequel l'élément de fil (29) est formé d'une fibre à haute résistance dont la résistance à la rupture par traction est supérieure ou égale à 2GPa et un module d'élasticité est supérieure ou égal à 50 GPa.
